(19) Europäisches Patentamt
European Patent Office
Office européen des brevets

(11) Publication number: **0 193 154 B1**

(12) **EUROPEAN PATENT SPECIFICATION**

(45) Date of publication of patent specification: **29.01.92**

(51) Int. Cl.[5]: **G01N 27/30**, G01N 27/40, //G01N33/543

(21) Application number: **86102363.8**

(22) Date of filing: **24.02.86**

(54) **Potential-causing element for immunosensor.**

(30) Priority: **25.02.85 JP 36839/85**

(43) Date of publication of application:
**03.09.86 Bulletin 86/36**

(45) Publication of the grant of the patent:
**29.01.92 Bulletin 92/05**

(84) Designated Contracting States:
**AT BE CH DE FR GB IT LI NL SE**

(56) References cited:
**FR-A- 2 384 789**
**FR-A- 2 476 125**
**GB-A- 2 052 046**
**US-A- 4 334 880**

**JOURNAL OF THE ELECTROCHEMICAL SOCIETY, vol. 130, no. 10, October 1983, pages 2042-2044, Manchester, New Hampshire, US; G. TOURILLON et al.: "Stability of conducting polythiophene and derivatives"**

(73) Proprietor: **Juridical Foundation The Chemo-Sero-Therapeutic Research Institute**
**668, Okubo Shimizu-machi**
**Kumamoto-shi Kumamoto-ken(JP)**

(72) Inventor: **Taniguchi, Isao**
**14-31, Kita-jutaku Higashi-machi**
**Kumamoto-shi Kumamoto-ken(JP)**
Inventor: **Yasukouchi, Kazuo**
**1-6-54, Hakuzan**
**Kumamoto-shi Kumamoto-ken(JP)**
Inventor: **Tsuji, Ichiroh**
**5-18-27, Wakaba**
**Kumamoto-shi Kumamoto-ken(JP)**

(74) Representative: **Vossius & Partner**
**Siebertstrasse 4 P.O. Box 86 07 67**
**W-8000 München 86(DE)**

**EP 0 193 154 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an immunosensor and more particularly to a potential-causing element for use in an immunosensor.

Immunosensors are used to determine relatively large proteins or polypeptides through an antigen-antibody reaction. In a typical analysis with an immunosensor, a potential-causing element (electrode or membrane) having an antigen (or an antibody) thereon is immersed in a solution containing a corresponding antibody (or antigen). A potential (electric-potential) caused across the electrode or the membrane due to the formation of an antigen-antibody complex is detected to determine qualitatively or quantitatively the presence of the corresponding antibody (or the antigen).

The immunosensor potential-causing electrode as mentioned above has typically been prepared in one of the following manners:

1) Through chemical bonds such as covalent bond, an antigen (or an antibody) is bound directly onto the surface of an electrode body made of a metal or the like.
2) A polymer film, onto which an antigen (or an antibody) is bound, is coated over an electrode body.

The former method can be carried out by treating titanium wire or tungsten wire, as the electrode body, with an acid such as hydrochloric acid so as to form an oxide-film thereon, followed by a treatment with an aqueous solution containing cyan-bromide. The resultant electrode is then immersed in an antigen- or antibody-containing solution to bind or fix the antigen or antibody thereon. In the latter method, the surface of the electrode body is coated with a film of a polymer, such as cellulose acetate, sephadex, polystyrene or polyacrylamide, by means of solution-coating, covalent bonding, electrodeposition or vaporization techniques. An antigen or an antibody is then fixed onto the surface of the coated electrode body.

An immunosensor potential-causing element can also be used in the form of a membrane. Such a membrane is typically prepared by coating a glass plate with a solution containing triacetyl cellulose or collagen. The resultant film of triacetyl cellulose or collagen is removed from the glass plate and then subjected to a treatment by which an antigen or an antibody is bound or fixed onto the triacetyl cellulose or the collagen film.

As can be seen from the above, the conventional methods require rather sophisticated techniques for obtaining the electrodes or the membranes for immunosensors. Particularly, it is hard with such methods to control the processing parameters so as to obtain a membrane or an electrode which will develop the desired immunoresponse suitable for use in an immunosensor.

US-A-4 334 880 describes an analytical device with a semiconductive polyacetylene element. The polyacetylene of the element is associated with a binding substance having specific affinity for the analyte. The semiconductive properties of polyacetylene are utilized for determining the concentration of the analyte. In the presence of the analyte, the semiconductive element varies its resistance and in order to measure the respective resistance, an external voltage is applied.

Journal of the Electrochemical Society, vol. 130, no. 10, October 1983, pp. 2042-2044, Manchester, New Hampshire, US; G. Tourillon et al.: "Stability of conducting polythiophene and derivatives" describes the preparation of conductive polymers, such as polythiophene, polyfuran, and derivatives which appear as interesting candidates for organic electrodes for energy storage or display.

Document D3 (FR-A-2 384 789) describes the use of dialdehydes and an amine in the fixation of biological binding substances to a polymer.

The primary object of the present invention is to provide a novel potential-causing element for use in immunosensor, which can be prepared in a relatively easy manner to obtain desired immunoresponse characteristics.

The present inventors have found that this object can be accomplished by the use of polypyrrole or polythiophene as the polymer film onto which an antigen or an antibody is bound.

Polypyrrole and polythiophene are polymers having the following repeating units, respectively.

Polypyrrole:

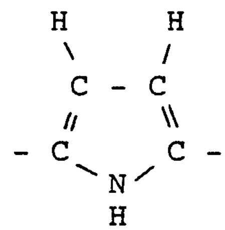

Polythiophene:

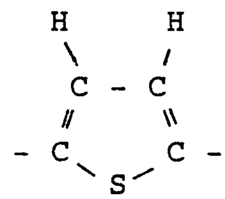

While these polymers are known as functional polymers which will exhibit photoresponse or electrical conductivity, no disclosures or suggestions have been found that they can be used as materials for electrodes or membranes for immunosensors. The present inventors perceived that polypyrrole or polythiophene can be prepared by means of electrolytic polymerisation, in which the operating parameters can be easily controlled to produce polypyrrole or polythiophene film suitable for use as the immunosensor element when an antigen or an antibody is bound thereon. Thus, it has been found by the present inventors that polypyrrole or polythiophene will provide highly sensitive membrane or electrodes for immunosensors, particularly when produced as a film having a specific range of thickness.

The present invention therefore provides a potential-causing electrode for use in immunosensors, comprising a film of polypyrrole or polythiophene bound with an antigen or an antibody and an electrode body overcoated with said film, in which said polypyrrole film or polythiophene film preferably has a thickness in the range of about 0.05 μm to about 0.25 μm.

There is further provided according to the present invention a potential-causing element, in the form of a membrane, for use in an immunosensor, comprising a film of polypyrrole or polythiophene bound with an antigen or an antibody, in which said polypyrrole film or polythiophene film preferably has a thickness in the range of about 0.5 μm to about 5 μm.

The potential-causing element according to the present invention can be prepared through electrolytic polymerization (electrochemical polymerization) of pyrrole or thiophene, by the process as described below, which is another aspect of the present invention.

For the electrolytic polymerization pyrrole or thiophene is dissolved in a solvent to form a solution. The solvent is a polar solvent which will dissolve pyrrole or thiophene (monomer) but will not dissolve polypyrrole or polythiophene (polymer). Such solvents may be exemplified by nitriles such as acetonitrile and benzonitriles; amides such as dimethyl formamide; amines such as pyridine; ethers such as tetrahydrofuran and 1,4-dioxane; acids such as acetic acid; alcohols such as methanol and ethanol. There may also be included such solvents as propylene carbonate, nitromethane, methylene chloride, acetone, methylethyl ketane, water and the like. Pyrrole or thiophene is dissolved in one or more of these solvents, in a concentration generally in the range of 20 to 200 milimoles/l.

In forming the electrolytic polymerization solution, it is preferable, depending upon the type of solvents, to add an electrolyt such as tetraalkylammonium-boron-tetrafluoride, tetraalkylammonium perchlorate, tetraalkylammonium-phosphorus-hexafluoride, and tetraalkyl hydrogensulfate, in which the alkyls have 1 to 10 carbon atoms. For example, when a nitrile such as acetonitrile is used as solvent, it is preferable to add tetraalkylammonium-boron-tetrafluoride, tetraalkylammonium perchlorate or the like in an amount of 50 to 100 milimoles/l. 1,4-Dioxane, propylene carbonate, acetone or nitromethane is used concurrently with the addition of tetrabutylammonium-boron-tetrafluoride or tetraethylammonium perchlorate in an amount of 50 to 100 milimoles/l. When a relatively conductive solvent such as water is used, the addition of an ordinary inorganic salt, a buffer solution and tetraethylammonium is preferred.

In the electrolytic polymerisation solution containing pyrrole or thiophene thus prepared, there are placed a working electrode (an electrode on which the polypyrrole film or polythiophene film is to be formed) and a counter electrode, while a reference electrode (e.g. $Ag/AgClO_4$ electrode) may also be concurrently used. As materials for the working electrode, there may be mentioned metals such as

platinum, aluminum and gold; metal oxides such as tin oxide and titanium oxide; semiconductive substances such as silicone and gallium arsenide; and carbonaceous substances such as graphite and glassy carbon. These electrodes can be used in any shape known in the art.

The electrolytic polymerization of pyrrole or thiophene is carried out by the passage of electricity through the solution between the electrodes, preferably with oxygen dissolved in the solution having been removed, and most preferably under an atmosphere of nitrogen. Immediately after the passage of electricity is started, the film of polypyrrole or polythiophene (black in color) starts to be formed on the working electrode (electrode body).

One of the notable advantages of the present invention, in which a polymer film (polypyrrole film or polythiophene film) is prepared through electrolytic polymerization, is that the thickness of the film can be easily controlled so as to obtain an immunosensor potential-causing element which will exhibit desired immunoresponse characteristics. Thus, the film thickness or the film formation rate is determined by electric charge and current density, depending upon the type of solvent used and other operating parameters. For example, in a case where acetonitrile is used as solvent, a polymer film of a thickness of about 50 to 250 nm is produced by the electric charge of 20 to 100 $mC/cm^2$ at a current density of 20 to 100 $\mu A/cm^2$, while the use of an aqueous solvent for producing a polymer film of the same thickness requires about ten times as much current density and about 5 to 20 times as much electric charge. In a case where an electrolytic polymerization solution is added with a fixation-improving (binding-improving) agent as described later, a higher current density and a higher electric charge are required for producing a polymer film of the same degree of thickness.

For the purpose of improving the physical strength or workability of the polypyrrole film or the polythiophene film, the monomer-containing solution for the electrolytic polymerization may be added with film-strengthening agent(s) such as acetyl cellulose (e.g. bromoacetyl cellulose or triacetyl cellulose) in an amount of about 1 to 2%. In such case, the solvent system must be so selected that it will sufficiently dissolve the film-strengthening agent as well as pyrrole or thiophene. For example, when triacetyl cellulose is used, a mixture of solvents, such as acetonitrile plus methylene chloride (3:2), is preferably used.

The electrode body (working electrode) on which a polypyrrole film or a polythiophene film has been formed in the manner as described above is then immersed in a solution containing an antigen or an antibody for binding or fixing the antigen or the antibody thereon, following a washing operation. Alternatively, the polypyrrole film or the polythiophene film is separated or exfoliated from an electrode body, washed and then immersed in an antigen- or antibody-containing solution for obtaining a potential-causing element rather than a potential-causing electrode.

A preferred solution containing an antigen or an antibody is, for example, one which has been adjusted to pH 7.0 with a phosphate buffer. The operation of immersing the polymer film-formed electrode or the polymer film in the antigen- or antibody-containing solution must be carefully conducted so that the antigen or the antibody is bound or fixed onto the polymer film in a highly dense and homogeneous state in order to prevent the adsorption of any non-specific substance, i.e. reactions other than the desired antigen-antibody reaction which is to be detected by the immunosensor. The prevention of such non-specific adsorption may be enhanced by the treatment of the antigen-or antibody-fixed electrode or film (membrane) with a suitable agent such as BSA (bovine serum albumin).

The fixation or binding of an antigen or an antibody onto the polymer can be carried out concurrently with the polymerization of pyrrole or thiophene by adding in advance the antigen or the antibody into a pyrrole-or thiophene-containing solution for electrolytic polymerization where an aqueous solvent is used. In such case, there is no need of adding an electrolyte since the antigen or the antibody per se serves as an electrolyte. This procedure is advantageous in that the polypyrrole film or polythiophene film on which an antigen or an antibody is fixed can be produced in a single step.

It has been found that the fixation or binding of an antigen or an antibody onto the polymer is highly improved by a treatment with dialdehydes such as glyoxal, malondialdehyde, succinaldehyde, glutaraldehyde and adipinaldehyde, or amines such as allylamine, 1,8-octanediamine, 4-amino-methyl-1,8-octanediamine and hexamethylene diamine, and mixtures thereof. Thus, in a preferred embodiment according to the present invention, a polypyrrole film- or polythiophene film-coated electrode or a polypyrrole film or polythiophene film produced by the electrolytic polymerization is treated with a solution containing the dialdehyde or diamine as described above, prior to the immersion of such electrode or film into the antigen- or antibody-containing solution. Alternatively, the dialdehyde or amine may be present in the polymerization solution containing pyrrole or thiophene so that the treatment with such a dialdehyde or amine is carried out concurrently with the polymerization. The polypyrrole film or polythiophene film thus treated with the dialdehyde or diamine is found to have a highly improved fixation (binding) characteristic with an antigen or an antibody, thereby providing a potential-causing element (electrode or membrane) with a high immunoresponse.

If necessary, the polypyrrole film or polythiophene film, on which an antigen or an antibody is fixed or bound, can be treated with sodium hydrogen borate or the like so as to stabilize the fixation between the polymer and the antigen (or the antibody) and also improve the degree of freedom of the fixed antigen or antibody.

Any type of antigen or antibody can be bound or fixed onto the polypyrrole film or polythiophene film according to the present invention. Such antigens (or antibodies) include various immunoglobulins (IgG, IgM, IgA, etc.), anti-immunoglobulins, albumines, and hCG.

The antigen (or antibody) fixed-polypyrrole or polythiophene film prepared in the above-mentioned manner can be used as a potential-causing element for an immunosensor, in the form of an electrode in which an electrode body is coated with the antigen (or antibody) fixed-film, or in the form of a membrane in which the antigen (or antibody) fixed-film is used singly. The thickness of the potential-causing element in the form of a membrane is preferably about 10 to 20 times as large as that of the element in the form of an electrode, since it is difficult to exfoliate an extremely thin film from the electrode body. It has been found that polypyrrole film is more preferable than polythiophene film as the former is easier to be handled as a membrane and exhibits higher fixation characteristics with an antigen or an antibody.

Figure 1 illustrates a typical example of immunosensor system including an electrode 10 according to the present invention, which comprises an antigen (or an antibody) -bound polypyrrole or polythiophene film overcoating an electrode body, and a reference electrode 11. The reference electrode 11 is any suitable electrode used in the art, such as saturated calomel electrode and Ag/AgCl electrode. The working electrode 10 is immersed in an antibody (or an antigen) -containing solution 12 in a container 16. The solution 12 is connected with the electrode liquid of the reference electrode through a connecting tube (or bridge) 14 having a filter 13. Thus, the electric potential (more specifically, the change in the potential) caused across the working electrode 10 due to antigen-antibody reaction is detected by a voltmeter (not shown), while the solution 12 is preferably stirred with a stirring device such as a magnetic stirrer 15. The solution containing the antibody (or the antigen) is usually a saline solution, PBS or the like.

Figure 2 illustrates an example of immunosensor system where an antigen (or an antibody) -bound polypyrrole or polythiophene is used as it is, i.e. in the form of a membrane, for measuring the membrane potential (more specifically, the change in membrane potential) due to the antigen-antibody reaction. Thus, the system of Figure 2 includes the antigen (or the antibody) -bound polypyrrole or polythiophene membrane 21 provided between two saturated calomel electrodes 23, 24 as reference electrodes. The membrane 21 is mounted on a glass tube 28 containing a dilution liquid (a saline solution or PBS) 29, the concentration of which is about one tenth of that of a saline solution or PBS used to prepare a solution containing an antibody (or an antigen) to be detected. The electrode 23 is connected with the liquid 29 through a bridge 25 while the electrode 24 is connected with the solution 22 in a container 30 through a bridge 26. The measurement of a membrane potential across the membrane 21, preferably while stirring the solution 22 with a magnetic stirrer 31, with such system provides the detection of the aimed antibody (or the antigen) in the solution.

Examples will be given below for better understanding of the present invention.

## Example 1

A solution for electrolytic polymerization was prepared by dissolving pyrrole and tetraethylammonium-boron-tetrafluoride, each being at the concentration of 0.1 mole/l, in acetonitrile. In the solution thus prepared are immersed a working electrode made of platinum wire (1 mm in diameter and 4 mm in length) and a counter electrode made of platinum plate. The passages of electricity under an atmosphere at the current density of 20 $\mu A/cm^2$ with varying electric charge resulted in platinum electrodes overcoated with polypyrrole film of varying thickness which was measured by an electron microscope. Such film thickness can be expressed in terms of electric charge ($mC/cm^2$) as shown in Figure 3, where the electric charge of 80 $mC/cm^2$ was found to correspond to the thickness of about 200 nm (0.2 $\mu m$) under this process condition.

Each of the polypyrrole film-overcoated electrodes was immersed for 20 minutes in a solution containing anti-IgG at the concentration of $2.32 \times 10^{-3}$ mg/ml so as to fix the anti-IgG on the polypyrrole film.

The change in electrode potential ($-\Delta E$/mV) was measured before and after the fixation of the anti-IgG, the change being a criterion or measure of the degree of the fixation. The results were given in Figure 3, which demonstrates that the polypyrrole film highly adsorbs the anti-IgG. It is particularly noted that the film having a thickness of about 50 to 250 nm (corresponding to the electric charge of 20 to 100 $mC/cm^2$)

exhibits a good response.

Example 2

With the same solution for electrolytic polymerization as used in Example 1, six electrodes were prepared by forming a polypyrrole film on working electrodes of platinum wire (each being 1 mm in diameter and 6 mm in length), followed by the fixation of IgG on the film, under the conditions described below:

(1) The electrolytic polymerization was carried out at a current density of 100 $\mu A/cm^2$ with an electric charge of 60 $mC/cm^2$ (20 $\mu A$, 10 minutes) to form a polypyrrole film on the platinum wire, which was washed with acetonitrile and then with water. The resultant polypyrrole film-formed electrode was immersed in a solution containing IgG at a concentration of 150 mg/ml for 3.5 days and then in 1% BSA for two days. The polypyrrole film-overcoated platinum electrode with the IgG being fixed on the film (film thickness: 150 nm) thus prepared was designated as Electrode A.

(2) A polypyrrole film was formed on the platinum wire through electrolytic polymerization and the film was washed, in the same manner as in (1). The resultant film-overcoated electrode was immersed in 4-aminomethyl-1,8-octanediamine for four days, 5% glutaraldehyde aqueous solution for one day, a solution containing IgG at the concentration of 150 mg/ml for seven days, and then 5% BSA for five days to obtain a polypyrrole film-overcoated platinum electrode with IgG fixed on the film (Electrode B).

(3) To a solution for electrolytic polymerization as in (1) was added allylamine at 0.1 mole/l. The electrolytic polymerization was carried out at a current density of 25 $mA/cm^2$ with an electric charge of 40 $C/cm^2$ (5 mA, one hour) to form a polypyrrole film (thickness: 160 nm) on the platinum wire, followed by washing as in (1). The resultant electrode was immersed in a solution containing IgG at 150 mg/ml for three days and then in 5% BSA for two days to obtain a polypyrrole film-overcoated platinum electrode with IgG fixed on the film (Electrode C).

(4) To a solution for electrolytic polymerisation as in (1) was added glutaraldehyde at a concentration of 5%. The electrolytic polymerization was carried out at a current density of 100 $\mu A/cm^2$ with an electric charge of 300 $mC/cm^2$ (20 $\mu A$, 50 minutes) to form a polypyrrole film (thickness: 150nm) on the platinum wire. The film thus formed was washed in the same manner as in (1), and then the film-coated electrode was immersed in a solution containing IgG at a concentration of 150 mg/ml for two days and 5% BSA for two days to obtain a IgG-fixed and polypyrrole film-overcoated platinum electrode (Electrode E).

(5) A solution for electrolytic polymerization containing 5% of glutaraldehyde as used in (4) was subjected to electrolytic polymerization at a current density of 100 $\mu A/cm^2$ and an electric charge of 360 $mC/cm^2$ (20 $\mu A$, 60 minutes) to form a polypyrrole film having a thickness of 150 nm, which was washed as in (1). The resultant film-overcoated electrode was immersed in 4-aminomethyl-1,8-octanediamine for four days, 50% glutaraldehyde aqueous solution for one day, a solution containing IgG at 150 mg/ml for two days and then 5% BSA for five days to obtain a polypyrrole film-overcoated platinum electrode with IgG being fixed on the film (Electrode E).

(6) To a solution for electrolytic polymerization as in (1), there was added glutaraldehyde at a concentration of 10%. The electrolytic polymerization was carried out at a current density of 100 $\mu A/cm^2$ and an electric charge of 720 $mC/cm^2$(20 $\mu A$, 2 hours) and further at a current density of 250 $\mu A/cm^2$ and an electric charge of 900 $mC/cm^2$ (50 $\mu A$, one hour) to form a polypyrrole film on the platinum wire electrode, followed by washing the film as in (1). The resultant electrode was then immersed in an IgG-containing solution (at 150 mg/ml) for two days and in 0.1 N $NaBH_4$ (pH 7.8) for one day to obtain a polypyrrole film-overcoated platimun electrode with the IgG being fixed onto the film (Electrode F).

Each of the Electrodes A through F was immersed, as working electrode in the immunosensor system as shown by Figure 1, in a solution containing anti-IgG at the concentration of $8.52 \times 10^{-3}$ mg/ml. Measurement was made, with each Electrode, of the potential (more strictly, the change in electric potential), $-\Delta E$/mV, caused while the Electrode was immersed in the anti-IgG containing solution. The results are given in Figure 4, which demonstrates that all the Electrodes exhibit immunoresponse. It is particularly noted that the Electrodes B, C, D, E and F (which have been prepared by the methods including the fixation-improving treatment with amine or dialdehyde) exhibit remarkable immunoresponse characteristics.

Example 3

The immunoresponse of Electrode F was measured with anti-IgG-containing solutions with varying concentrations of the anti-IgG, in the manner as described in Example 2. The results are given in Fig. 5,

which demonstrates that the detection of anti-IgG can be sufficiently made as low as about $10^{-5}$ mg/ml by using the electrode according to the present invention. The results are also summarized in Fig. 6 in terms of the potential change after 20 minutes against the logarithmic concentration of anti-IgG, which provides a good linearity. It was found that a linearity for calibration can also be obtained with respect to other lapses of time after the immersion of the electrode in the solution.

Example 4

A solution for electrolytic polymerization was prepared by adding IgG in an aqueous solutin saturated with pyrrole, so that the concentration of IgG was 15 mg/ml. In the polymerization solution thus prepared was immersed a platinum electrode (0.5 mm in diameter and 5.8 mm in length) as a working electrode. Electrolytic polymerization was conducted under the atmosphere of nitrogen at a current density of 1 mA/cm2 and an electric charge of 400 mC/cm$^2$ (100 $\mu$A, six minutes) to obtain a polypyrrole film-coated platinum electrode with the IgG being fixed on the film where the thickness of the film is 100 nm (0.1 $\mu$). The electrode thus obtained was used as a working electrode in the immunosensor system as illustrated in Fig. 1 to determine the immunoresponse to anti-IgG-containing solutions of varying anti-IgG concentrations, with satisfactory results as shown in Fig. 7.

Example 5

With an electrolytic polymerization solution as used in Example 1, electrolytic polymerization was carried out at an electric charge of 1.6 C/cm$^2$ to form a polypyrrole film on a platinum plate, followed by washing with acetonitrile or in distilled water to obtain a membrane having a thickness of 4 $\mu$m. The membrane was treated with a mixture of 4-aminomethyl-1,8-octanediamine and 50% aqueous solution of glutaraldehyde (10:1 by volume) for one day and then immersed in a solution containing IgG at a concentration of 150 mg/ml for two days so as to fully fix the IgG on the membrane. The membrane after the immersion was used as a working membrane in the immunosensor system as illustrated in Fig. 2 to determine its immunoresponse to anti-IgG. As a result, there was observed a clear change in electric potential, which fact indicates a high adaptability of the membrane to the immunosensor.

For comparison, measurement of membrane potential was done with an IgG-fixed membrane made of triacetyl cellulose which has been prepared by the conventional technique as mentioned previously, with the result that there was observed an extremely low response, i.e. about one tenth as low as that with the membrane of the present invention.

Example 6

An IgG-fixed polythiophene membrane was prepared by using thiophene instead of pyrrole in Example 5. The IgG-fixed polythiophene membrane was found to exhibit a good response to anti-IgG (although slightly lover than the polypyrrole membrane in Example 5).

## Claims

**1.** A potential-causing electrode for use in immunosensors, comprising a film of polypyrrole or polythiophene bound with an antigen or an antibody and an electrode body overcoated with said film, in which said polypyrrole or polythiophene film has a thickness in the range of about 0.05 $\mu$m to about 0.25 $\mu$m.

**2.** A potential-causing membrane for use in immunosensors, comprising a film of polypyrrol or polythiophene bound with an antigen or an antibody, in which said polypyrrole or polythiophene film has a thickness in the range of about 0.5 $\mu$m to about 5 $\mu$m.

**3.** A process for producing a potential-causing element according to Claim 1 or 2 for use in immunosensors, which comprises preparing a solution for electrolytic polymerization by dissolving pyrrole or thiophene in a solvent, placing a working electrode and a counter electrode in said solution for electrolytic polymerisation, conducting electrolytic polymerization of said solution containing the pyrrole or thiophene by passing electricity through the solution between said electrodes under a controlled current density and electric charge so as to form a polypyrrole or polythiophene film of a desired thickness on said working electrode, and fixing an antigen or an antibody on said polypyrrole or

polythiophene film.

4. The process as claimed in claim 3 which further includes the step of exfoliating the polypyrrole or polythiophene film from the working electrode so as to provide the potential-causing element in the form of a membrane.

5. The process as claimed in claim 3 in which the step of fixing the antigen or the antibody on the polypyrrole or polythiophene film is carried out, after the electrolytic polymerization, by immersing the working electrode or the membrane, on which the polypyrrole or polythiophene film has been formed, in an antigen- or antibody-containing solution.

6. The process as claimed in claim 3 in which the step of fixing the antigen or the antibody on the polypyrrole or polythiophene film is carried out concurrently with the electrolytic polymerization of pyrrole or thiophene by in advance adding the antigen or the antibody to the pyrrole- or thiophene-containing solution for electrolytic polymerization.

7. The process as claimed in claim 3 which further includes the step of treating the polypyrrole or polythiophene film with a dialdehyde selected from glyoxal, malondialdehyde, succinaldehyde, glutaraldehyde, adipinaldehyde and a mixture thereof, or an amine selected from allylamine, 1,8-octanediamine, 4-amino-methyl-1,8-octanediamine, hexamethylene diamine and a mixture therof.

8. The process as claimed in claim 7 in which the treatment with the dialdehyde or the amine is carried out prior to the immersion of the electrode or the membrane in the antigen- or antibody-containing solution.

9. The process as claimed in claim 7 in which the treatment with the dialdehyde or the amine is carried out concurrently with the electrolytic polymerization by adding in advance said dialdehyde or amine to the solution for elctrolytic polymerization.

## Revendications

1. Electrode génératrice de potentiel utilisable dans des immunocapteurs, comprenant un film de polypyrrole ou de polythiophène lié à un antigène ou à un anticorps et un corps d'électrode revêtu de ce film, dans laquelle ledit film de polypyrrole ou de polythiophène a une épaisseur située dans la gamme d'environ 0,05 $\mu$m à environ 0,25 $\mu$m.

2. Membrane génératrice de potentiel utilisable dans des immunocapteurs, comprenant un film de polypyrrole ou de polythiophène lié à un antigène ou à un anticorps, dans laquelle ledit film de polypyrrole ou de polythiophène a une épaisseur située dans la gamme d'environ 0,5 $\mu$m à environ 5 $\mu$m.

3. Procédé de production d'un élément générateur de potentiel selon la revendication 1 ou 2, utilisable dans des immunocapteurs, qui comprend la préparation d'une solution pour polymérisation électrolytique par dissolution de pyrrole ou de thiophène dans un solvant, l'immersion d'une électrode active et d'une contre-électrode dans ladite solution pour polymérisation électrolytique, la réalisation d'une polymérisation électrolytique de ladite solution contenant le pyrrole ou le thiophène par passage de courant dans la solution entre lesdites électrodes à une densité de courant et sous une charge électrique contrôlées de manière à former un film de polypyrrole ou de polythiophène d'une épaisseur souhaitée sur ladite électrode active, et la fixation d'un antigène ou d'un anticorps sur ledit film de polypyrrole ou de polythiophène.

4. Procédé selon la revendication 3, comprenant en outre l'étape de décollement du film de polypyrrole ou de polythiophène de l'électrode active de manière à obtenir l'élément générateur de potentiel sous forme d'une membrane.

5. Procédé selon la revendication 3, dans lequel l'étape de fixation de l'antigène ou de l'anticorps sur le film de polypyrrole ou de polythiophène est effectuée, après la polymérisation électrolytique, par immersion de l'électrode active ou de la membrane, sur laquelle le film de polypyrrole ou de

polythiophène a été formé, dans une solution contenant un antigène ou un anticorps.

6. Procédé selon la revendication 3, dans lequel l'étape de fixation de l'antigène ou de l'anticorps sur le film de polypyrrole ou de polythiophène est accomplie en même temps que la polymérisation électrolytique du pyrrole ou du thiophène par addition préalable de l'antigène ou de l'anticorps à la solution pour polymérisation électrolytique contenant du pyrrole ou du thiophène.

7. Procédé selon la revendication 3, qui comprend en outre l'étape de traitement du film de polypyrrole ou de polythiophène avec un dialdéhyde choisi parmi le glyoxal, le dialdéhyde malonique, l'aldéhyde succinique, le glutaraldéhyde, l'aldéhyde adipique et un mélange de ceux-ci, ou avec une amine choisie parmi l'allylamine, la 1,8-octanediamine, la 4-amino-méthyl-1,8-octanediamine, l'hexaméthylène diamine et un mélange de celles-ci.

8. Procédé selon la revendication 7, dans lequel le traitement avec le dialdéhyde ou l'amine est effectué avant l'immersion de l'électrode ou de la membrane dans la solution contenant l'antigène ou l'anticorps.

9. Procédé selon la revendication 7, dans lequel le traitement avec le dialdéhyde ou l'amine est effectué en même temps que la polymérisation électrolytique par addition préalable dudit dialdéhyde ou de ladite amine à la solution pour polymérisation électrolytique.

## Patentansprüche

1. Potentialverursachende Elektrode für Immunosensoren, mit einem Film von Polypyrrol oder Polythiophen, der mit einem Antigen oder einem Antikörper verbunden ist und mit einem Elektrodenkörper, der mit dem Film umhüllt ist, wobei der Polypyrrol- oder Polythiophenfilm eine Dicke im Bereich von ungefähr 0,05 µm bis ungefähr 0,25 µm aufweist.

2. Potentialverursachende Membran für Immunosensoren, mit einem Film aus Polypyrrol oder Polythiophen, der mit einem Antigen oder einem Antikörper verbunden ist, wobei der Polypyrrol- oder Polythiophenfilm eine Dicke im Bereich von ungefähr 0,5 µm bis ungefähr 5 µm aufweist.

3. Verfahren zum Herstellen eines potentialverursachenden Elements nach Anspruch 1 oder 2 für Immunosensoren, mit den Schritten: Herstellen einer Lösung für die elektrolytische Polymerisation durch Lösen von Pyrrol oder Thiophen in einem Lösungsmittel, Anordnen einer Arbeitselektrode und einer Gegenelektrode in der Lösung für die elektrolytische Polymerisation, Durchführen der elektrolytischen Polymerisation der Pyrrol oder Thiophen enthaltenden Lösung, in dem Elektrizität durch die Lösung zwischen den Elektroden mit einer gesteuerten Stromdichte und elektrischen Ladung durchgeleitet wird, um einen Polypyrrol- oder Polythiophenfilm einer gewünschten Dicke auf der Arbeitselektrode auszubilden, und Anbringen eines Antigens oder eines Antikörpers auf dem Polypyrrol- oder Polythiophenfilm.

4. Verfahren nach Anspruch 3, ferner mit dem Schritt: Ablösen des Polypyrrol- oder Polythiophenfilms von der Arbeitselektrode, um das potentialverursachende Element in Form einer Membran zur Verfügung zu stellen.

5. Verfahren nach Anspruch 3, wobei der Schritt zum Anbringen des Antigens oder des Antikörpers auf dem Polypyrrol- oder Polythiophenfilm nach der elektrolytischen Polymerisation durch Eintauchen der Arbeitselektrode oder der Membran, auf der der Polypyrrol- oder Polythiophenfilm ausgebildet wurde, in eine Antigen oder Antikörper enthaltende Lösung ausgeführt wird.

6. Verfahren nach Anspruch 3, wobei der Schritt zum Anbringen des Antigens oder des Antikörpers auf dem Polypyrrol- oder Polythiophenfilm gleichzeitig mit der elektrolytischen Polymerisation von Pyrrol oder Thiophen durch vorheriges Zugeben des Antigens oder des Antikörpers zu der Pyrrol oder Thiophen enthaltenden Lösung für die elektrolytische Polymerisation ausgeführt wird.

7. Verfahren nach Anspruch 3, ferner mit dem Schritt: Behandeln des Polypyrrol- oder Polythiophenfilms mit einem Dialdehyd, das aus einer Gruppe, bestehend aus Glyoxal, Malondialdehyd, Succinaldehyd, Glutardialdehyd, Adipinaldehyd und einer Mischung davon, ausgewählt ist oder mit einem Amin, das

aus einer Gruppe, bestehend aus Allylamin, 1,8-Octandiamin, 4-Amino-methyl-1,8-octandiamin, Hexamethylendiamin und einer Mischung davon ausgewählt ist.

**8.** Verfahren nach Anspruch 7, wobei die Behandlung mit dem Dialdehyd oder dem Amin vor dem Eintauchen der Elektrode oder der Membran in die Antigen oder Antikörper enthaltende Lösung ausgeführt wird.

**9.** Verfahren nach Anspruch 7, wobei die Behandlung mit dem Dialdehyd oder dem Amin gleichzeitig mit der elektrolytischen Polymerisation durch vorheriges Zugeben des Dialdehyds oder des Amins zu der Lösung für die elektrolytische Polymerisation ausgeführt wird.

Fig. 1

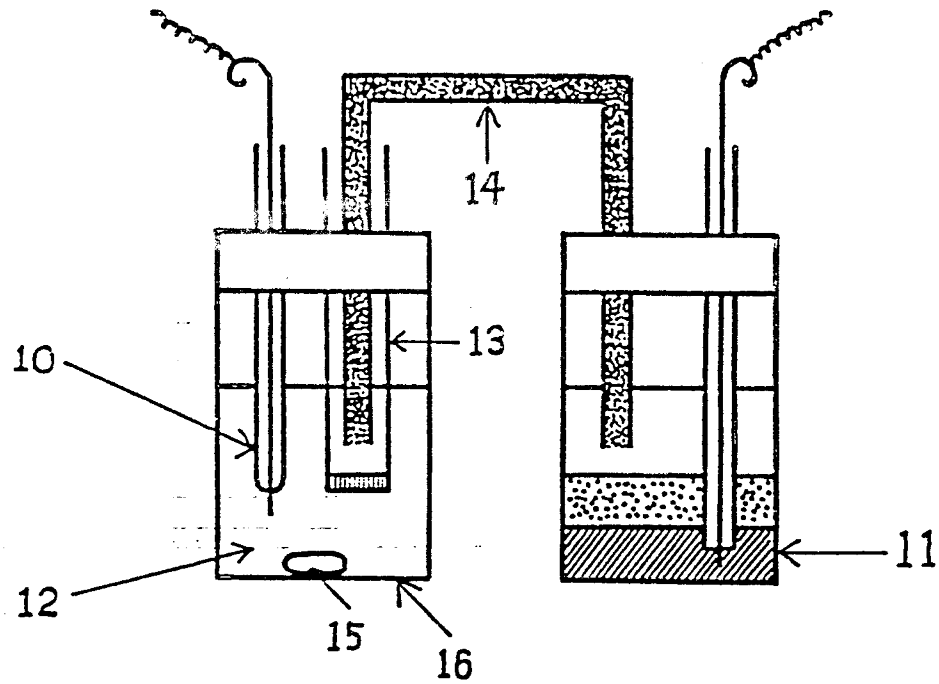


Fig. 2

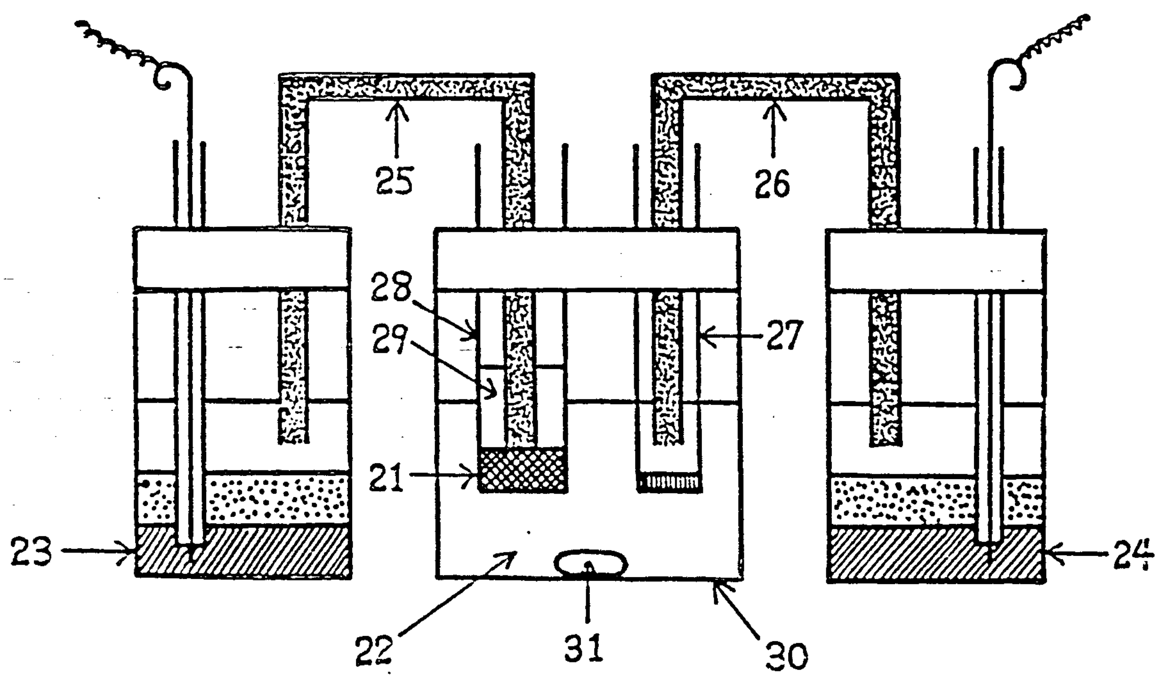

Fig. 3

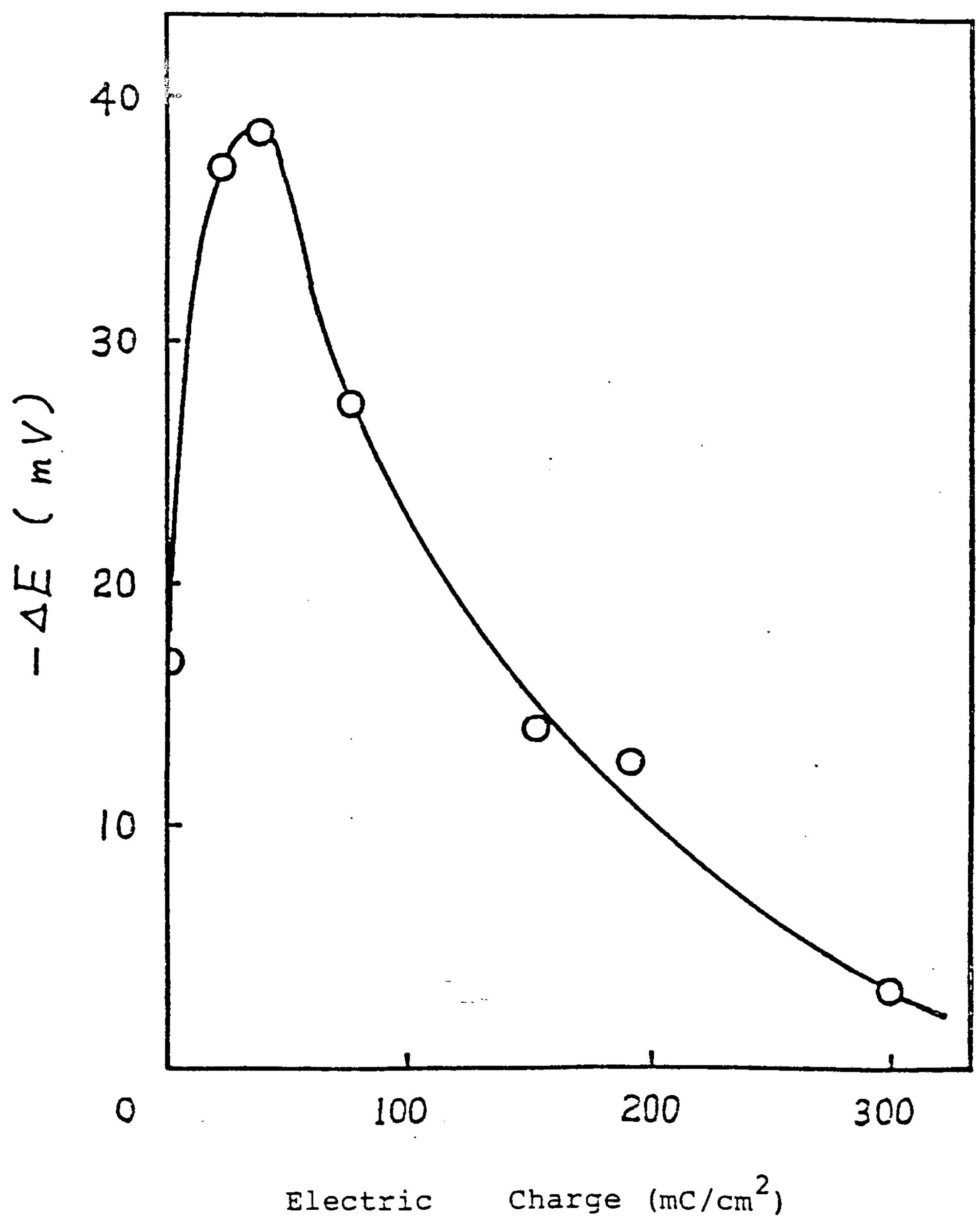

Fig. 4

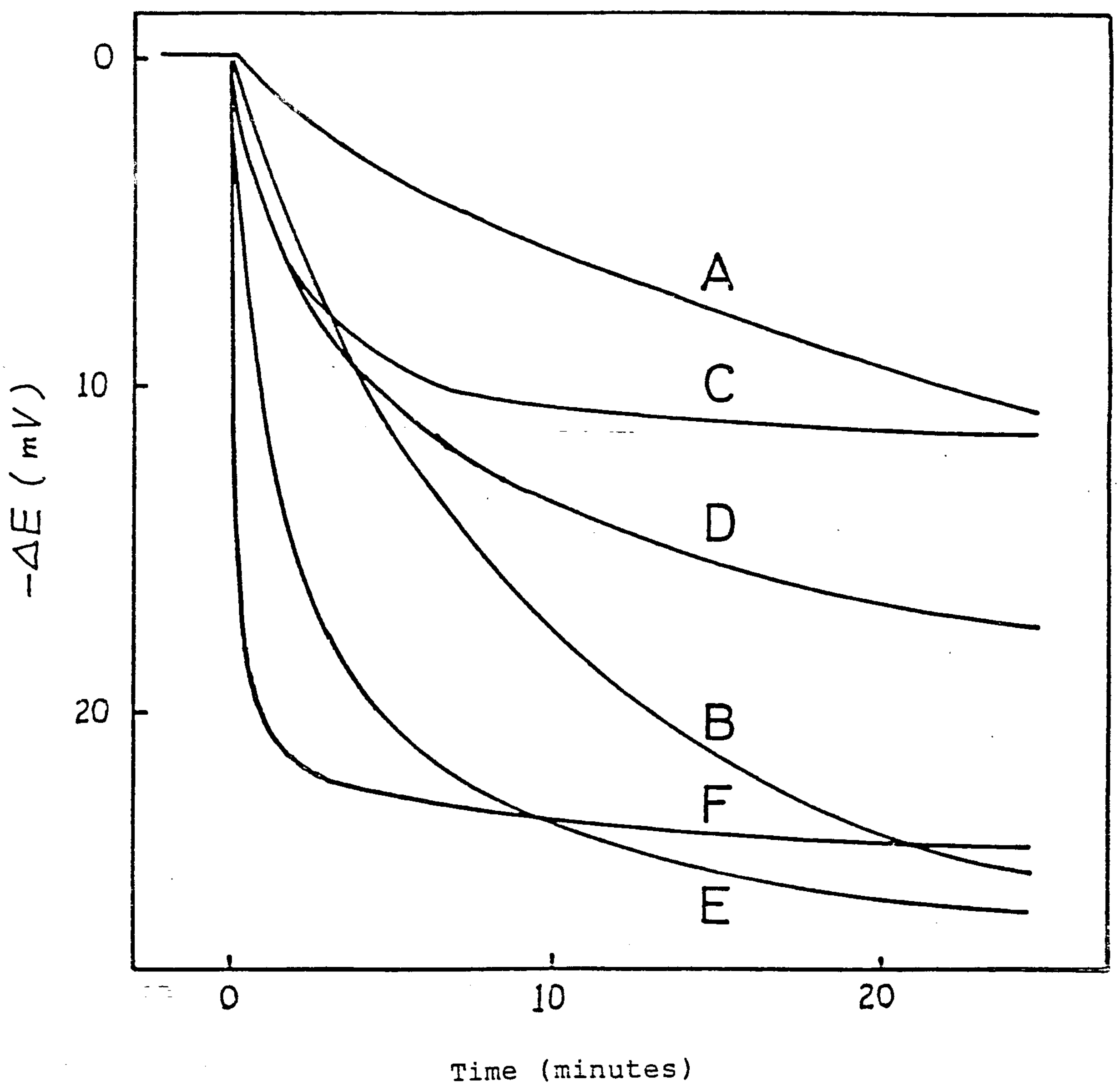

Fig. 5

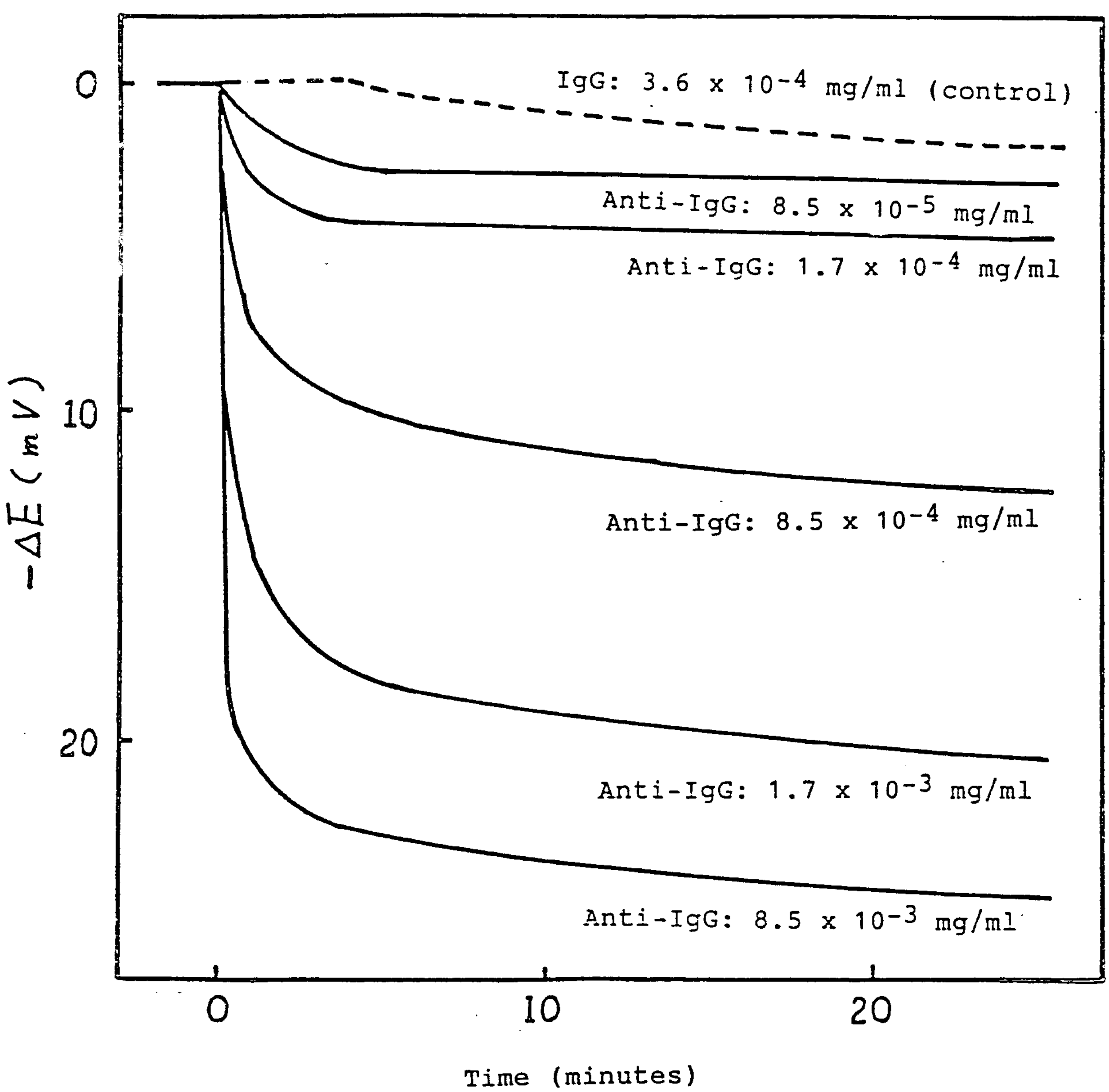

0
10
20
-ΔE (mV)
IgG: 3.6 x 10-4 mg/ml (control)
Anti-IgG: 8.5 x 10-5 mg/ml
Anti-IgG: 1.7 x 10-4 mg/ml
Anti-IgG: 8.5 x 10-4 mg/ml
Anti-IgG: 1.7 x 10-3 mg/ml
Anti-IgG: 8.5 x 10-3 mg/ml
0
10
20
Time (minutes)

Fig. 6

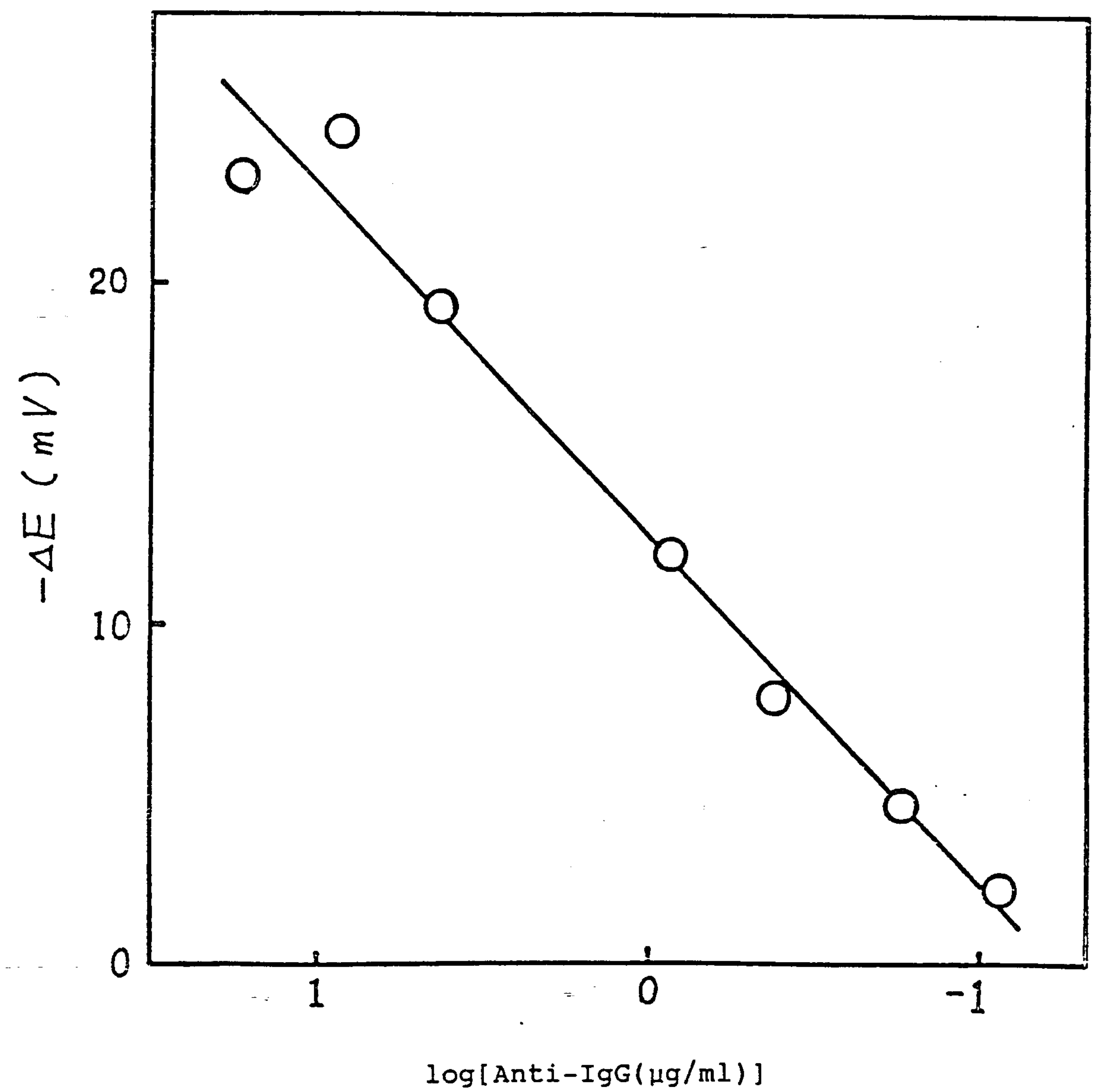

Fig. 7

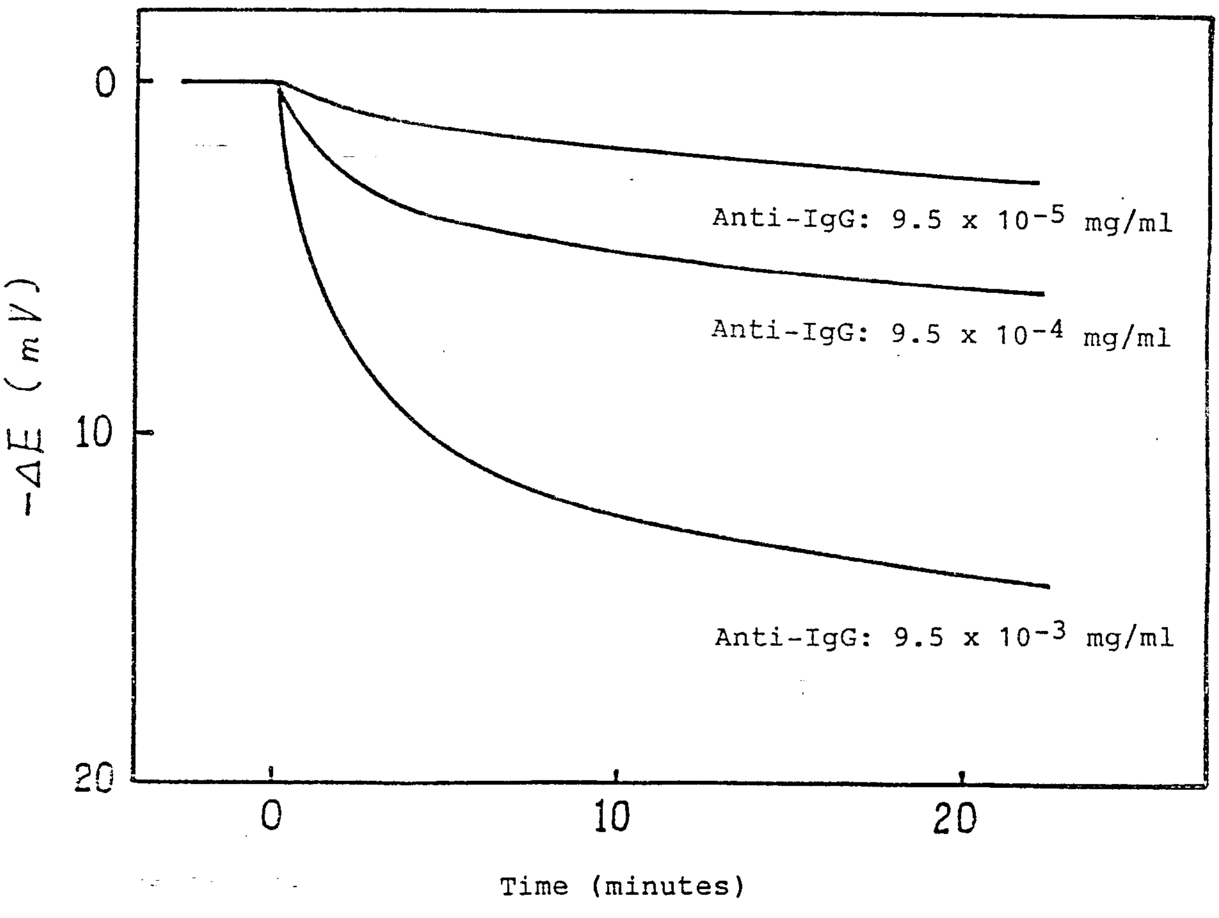
0
10
20
$-\Delta E$ ( mV )
Anti-IgG: 9.5 x $10^{-5}$ mg/ml
Anti-IgG: 9.5 x $10^{-4}$ mg/ml
Anti-IgG: 9.5 x $10^{-3}$ mg/ml
0
10
20
Time (minutes)